Europäisches Patentamt

European Patent Office

Office européen des brevets

(19)

(11) Veröffentlichungsnummer: **0 201 802**
**A2**

## (12) EUROPÄISCHE PATENTANMELDUNG

(21) Anmeldenummer: **86105910.3**

(22) Anmeldetag: **29.04.86**

(51) Int. Cl.⁴: **C07C 53/06 , C07C 51/41**

(30) Priorität: **09.05.85 DE 3516703**

(43) Veröffentlichungstag der Anmeldung:
**20.11.86 Patentblatt 86/47**

(84) Benannte Vertragsstaaten:
**AT BE CH DE FR GB IT LI LU NL SE**

(71) Anmelder: **RIEDEL-DE HAEN
AKTIENGESELLSCHAFT
Wunstorfer Strasse 40
D-3016 Seelze 1(DE)**

(72) Erfinder: **Schönfeld, Bernd, Dr.
Pappelweg 6
D-3052 Bad Nenndorf(DE)**

(74) Vertreter: **Beck, Bernhard
HOECHST AKTIENGESELLSCHAFT Zentrale
Patentabteilung Postfach 80 03 20
D-6230 Frankfurt/Main 80(DE)**

(54) **Wasserfreies basisches Aluminiumformiat.**

(57) Die Wasserlöslichkeit von Aluminiumsalzen der Ameisensäure ist unterschiedlich. Während Aluminiumtriformiat und Aluminiumdiformiat, das Kristallwasser enthält, in Wasser leicht löslich sind, ist die Wasserlöslichkeit von kristallwasserfreiem Aluminiumdiformiat nur sehr gering. Letzteres ist herstellbar durch Umsetzung eines Aluminiumsalzes einer anorganischen Säure, z.B. Aluminiumchlorid, mit Ameisensäure in wäßriger Lösung. Die Umsetzung erfolgt bei erhöhter Temperatur, und das wasserfreie basische Aluminiumformiat kristallisiert bereits in der heißen Reaktionslösung. Es eignet sich als Ausgangsmaterial zur Gewinnung von feinteiligem Aluminiumoxid.

EP 0 201 802 A2

Wasserfreies basisches Aluminiumformiat

Die Erfindung bezieht sich auf wasserfreies basisches Aluminiumformiat, Verfahren zur Herstellung von wasserfreiem basischen Aluminiumformiat durch Umsetzung eines Aluminiumsalzes einer anorganischen Säure mit Ameisensäure und Verwendung von wasserfreiem basischen Aluminiumformiat.

Verbindungen des Aluminiums mit Ameisensäure sind seit langem bekannt; sowohl Aluminiumtriformiat als auch kristallwasserhaltiges Aluminiumdiformiat sind Salze, die sich in Wasser leicht lösen (Gmelins Handbuch der anorganischen Chemie, Band 35, Al. Teil B 1, 1933, Seite 294). Ferner ist bekannt, daß schwerlösliche Salze des Aluminiums mit Ameisensäure durch Erhitzen von trockenem Tonerdehydrat mit Ameisensäure unter Ausschluß von Wasser hergestellt werden; dabei wird betont, daß sich ein basisches Aluminiumformiat der Formel Al(OH)(HCOO)$_2$ durch Kochen wäßriger Lösungen von neutralem Aluminiumformiat auch bei Zusatz größerer Mengen freier Ameisensäure nicht herstellen läßt (Deutsche Patentschrift 263 865).

Aufgabe der Erfindung ist die Bereitstellung eines in Wasser schwerlöslichen wasserfreien Aluminiumformiats, das durch Fällung aus einer wäßrigen Lösung herstellbar ist.

Die Erfindung betrifft ein wasserfreies basisches Aluminiumformiat der Formel Al(OH)(HCOO)$_2$.

Die Erfundung betrifft ferner ein Verfahren zur Herstellung von wasserfreiem basischen Aluminiumformiat durch Umsetzung eines Aluminiumsalzes einer anorganischen Säure mit Ameisensäure, das dadurch gekennzeichnet ist, daß eine wäßrige Lösung, die ein wasserlösliches Aluminiumsalz einer anorganischen Säure, Ammoniak und Ameisensäure enthält, wobei die Ammoniak-Menge 2,5 bis 4,0 mol und die Ameisensäure-Menge 2 bis 3 mol (jeweils bezogen auf 1 mol Aluminiumsalz) beträgt, auf eine Temperatur von 70 bis 100°C erhitzt und der entstandene kristalline Niederschlag nach Abkühlen des Reaktionsgemisches auf eine Temperatur unterhalb 30°C abgetrennt wird.

Weiterhin betrifft die Erfindung die Verwendung von wasserfreiem basischen Aluminiumformiat als Ausgangsmaterial zur Herstellung von feinteiligem Aluminiumoxid.

Als Ausgangsmaterial zur Herstellung des erfindungsgemäßen wasserfreien basischen Aluminiumformiats Al(OH)(HCOO)$_2$ wird ein Aluminiumsalz einer anorganischen Säure verwendet, das in Wasser leicht löslich ist. Besonders geeignete Salze sind z.B. Aluminiumbromid und Aluminiumchlorid-6-hydrat. Das wasserlösliche Aluminiumsalz wird in wäßriger Lösung in Gegenwart von Ammoniak mit Ameisensäure umgesetzt. Die Lösung zeigt einen pH-Wert von 3 bis 4, vorzugsweise von 3,3 bis 3,7. Die Umsetzung wird bei einer Temperatur von 70 bis 100°C, vorzugsweise 90 bis 100°C, durchgeführt. Die Ammoniak-Menge beträgt 2,5 bis 4,0 mol und die Ameisensäure-Menge 2 bis 3 mol (jeweils bezogen auf 1 mol Aluminiumsalz); ein Molverhältnis Aluminiumsalz/Ammoniak/Ameisensäure von 1: 3,5 : 3 ist besonders vorteilhaft. In der heißen Reaktionslösung entsteht ein kristalliner Niederschlag. Nach Abkühlen des Reaktionsgemisches auf eine Temperatur von unterhalb 30°, vorzugsweise unterhalb 25°C wird der kristalline Niederschlag von der Lösung abgetrennt, mit Wasser gewaschen und getrocknet. Das Abtrennen erfolgt vorzugsweise durch Filtrieren oder Zentrifugieren. Die Trocknung wird bei einer Temperatur von 100 bis 120°C durchgeführt. Das basische Aluminiumformiat fällt in Form eines feinkristallinen Pulvers an; die Ausbeute beträgt normalerweise mindestens 95 Prozent, vorzugsweise mindestens 98 Prozent.

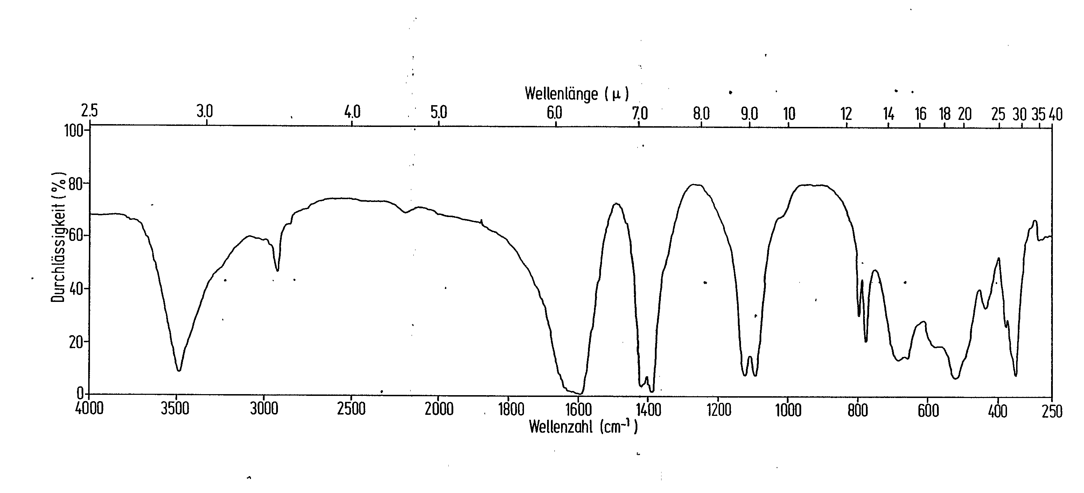

Das nach dem erfindungsgemäßen Verfahren erhältliche Aluminiumdiformiat enthielt kein Kristallwasser. Es ist durch eine hohe Reinheit ausgezeichnet; der Gehalt an Übergangsmetallen, insbesondere Eisen, ist sehr gering. Das IR-Spektrum - (KBr-Preßling) zeigt charakteristische Banden bei einer Wellenzahl von 800, 1100 und 1450 cm$^{-1}$. Das IR-Spektrum ist in Figur 1 wiedergegeben. Das Aluminiumdiformiat eignet sich vor allem als Ausgangsmaterial zur Herstellung von sehr reinem, feinteiligen Aluminiumoxid durch Glühen in oxidierender Atmosphäre bei einer Temperatur von etwa 800°C.

Das folgende Beispiel dient zur näheren Erläuterung der Erfindung. Prozentangaben beziehen sich jeweils auf das Gewicht.

Beispiel

In einem Emailkessel wurden zu einer Vorlage von 1 m$^3$ entmineralisiertem Wasser nacheinander 380 kg (1,55 kmol) Aluminiumchlorid-6-hydrat, 250 kg (4,6 kmol) 85prozentige Ameisensäure und 370 kg (5,4 kmol) 25prozentige Ammoniaklösung zugegeben. Durch Rühren des Gemisches entstand eine klare Lösung mit einem pH-Wert von 3,5, die 8 Stunden lang auf 100°C erwärmt wurde. Danach wurde das Reaktionsgemisch allmählich auf eine Temperatur von 20°C abgekühlt. Der bereits während des Erhitzens entstandene kristalline Niederschlag wurde durch Filtrieren abgetrennt, mit entmineralisiertem Wasser chloridfrei gewaschen und dann bei einer Temperatur von 105°C getrocknet. Es wurden 204 kg (=98 Prozent der Theorie) Aluminiumdiformiat in Form eines feinkristallinen Pulvers erhalten, dessen Gehalt an Eisen weniger als 5 ppm betrug.

**Ansprüche**

1. Wasserfreies basisches Aluminiumformiat der Formel Al(OH)(HCOO)$_2$.

2. Verfahren zur Herstellung von wasserfreiem basischen Aluminiumformiat durch Umsetzung eines Aluminiumsalzes einer anorganischen Säure mit Ameisensäure, dadurch gekennzeichnet, daß eine wäßrige Lösung, die ein wasserlösliches Aluminiumsalz einer anorganischen Säure, Ammoniak und Ameisensäure enthält, wobie die Ammoniak-Menge 2,5 bis 4,0 mol und die Ameisensäure-Menge 2 bis 3 mol (jeweils bezogen auf 1 mol Aluminiumsalz) beträgt, auf eine Temperatur von 70 bis 100°C erhitzt und der entstandene kristalline Niederschlag nach Abkühlen des Reaktionsgemisches auf eine Temperatur unterhalb 30°C abgetrennt wird.

3. Verwendung von wasserfreiem basischem Aluminiumformiat als Ausgangsmaterial zur Herstellung von feinteiligem Aluminiumoxid.
Patentansprüche für dem Vertragsstaat : AT

1. Verfahren zur Herstellung von wasserfreiem basischen Aluminiumformiat durch Umsetzung eines Aluminiumsalzes einer anorganischen Säure mit Ameisensäure, dadurch gekennzeichnet, daß eine wäßrige Lösung, die ein wasserlösliches Aluminiumsalz einer anorganischen Säure, Ammoniak und Ameisensäure enthält, wobie die Ammoniak-Menge 2,5 bis 4,0 mol die Ameisensäure-Menge 2 bis 3 mol (jeweils bezogen auf 1 mol Aluminiumsalz) beträgt, auf eine Temperatur von 70 bis 100°C erhitzt und der entstandene kristalline Niederschlag nach Abkühlen des Reaktionsgemisches auf eine Temperatur unterhalb 30°C abgetrennt wird.

2. Verwendung von wasserfreiem basischem Aluminiumformiat als Ausgangsmaterial zur Herstellung von feinteiligem Aluminiumoxid

0 201 802

Wellenlänge ( μ )

Durchlässigkeit (%)

Wellenzahl (cm⁻¹)